(19) Europäisches Patentamt
European Patent Office
Office européen des brevets

(11) **EP 4 079 272 A1**

(12) **EUROPEAN PATENT APPLICATION**

(43) Date of publication:
**26.10.2022 Bulletin 2022/43**

(21) Application number: **21170230.3**

(22) Date of filing: **23.04.2021**

(51) International Patent Classification (IPC):
**A61F 13/15** *(2006.01)* **G05B 19/418** *(2006.01)*

(52) Cooperative Patent Classification (CPC):
**A61F 13/15772; G05B 19/41875**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Ontex BV**
  **9255 Buggenhout (BE)**
• **Ontex Group NV**
  **9320 Erembodegem (BE)**

(72) Inventors:
• **WEBER, Ainas**
  **53474 Bad Neuenahr-Ahrweiler (DE)**
• **HEEGE, Thomas**
  **56761 Düngenheim (DE)**
• **TORRES SAAVEDRA, Antonio**
  **56330 Kobern-Gondorf (DE)**

(74) Representative: **Saurat, Thibault**
  **Ontex BV**
  **Legal Department**
  **Korte Keppestraat 21**
  **9320 Erembodegem (BE)**

(54) **SYSTEMS AND METHODS FOR DETECTING AND PROCESSING ABSORBENT ARTICLE DATA IN A PRODUCTION LINE**

(57) A method for inspecting disposable absorbent articles, the method comprising: providing a controller comprising an inspection algorithm with a convolutional neural network; providing a sensor, preferably selected from the group consisting of an optical sensor, thermal sensor, and combinations thereof; the sensor being in data communication with the controller; providing a reference database, wherein the reference database comprises a plurality of pre-processed images of disposable absorbent articles and/or components thereof that have been labeled with one or more of: a feature or element of the article and/or component thereof; a non-acceptable defect within a feature or element of the article and/or component thereof; an acceptable defect within a feature or element of the article and/or component thereof; an acceptable faultless feature or element of the article and/or component thereof; and combinations thereof; and the method comprising the steps of: training the convolutional neural network through a plurality of iterations or epochs; optionally validating the training, wherein the training step is repeated until a mean average precision score (MAP) of at least 0.9 is attained according to the formula:

$$MAP = \frac{\sum_{q=1}^{Q} AP(q)}{Q}$$

where Q is the number of queries; advancing one or more substrates through a converting process along a machine direction to form an array of absorbent articles; creating a plurality of images of said array of absorbent articles with the sensor; transmitting the images from the sensor to the controller; determining at least one of the properties for the labeled component with the inspection algorithm; cutting the substrate into discrete articles; and based on the determination of the least one of the properties for the labeled component, executing a control action, wherein the control action is selected from the group of automatic rejection of one or more articles, automatic machine-setting adjustment, a warning signal for machine maintenance scheduling, and a machine stop command.

**(Cont. next page)**

**FIG. 1**

**Description**

**TECHNICAL FIELD**

The disclosure relates to systems and methods for manufacturing disposable absorbent articles, and more particularly, systems for detecting and processing absorbent article data in a production line. The systems and methods herein are particularly adapted to detect and process absorbent article information in a holistic manner (i.e. by analysing the absorbent articles as a whole or as pluri-component sub-assemblies of said articles rather than individual components of said articles) and to optionally re-create a virtual copy of the physical article, compare said virtual copy with a database of virtual copies of other physical articles to discern characteristics of one or more components and/or sub-assemblies of said articles and to automatically generate signals to trigger a direct response to one or more manufacturing and/or rejection steps witin the manufacturing process.

More preferably, systems and methods herein make use of one or more algorithms generated with a neural network, preferably a convolutional neural network.

Absorbent articles herein are preferably selected from the group consisting of diapers (whether for baby or adults), pants (whether for baby or adults), pantiliners, briefs, sanitary napkins, and combinations thereof.

**BACKGROUND**

Along an assembly line, diapers and various types of other absorbent articles may be assembled by adding components to and otherwise modifying an advancing, continuous web of material. For example, in some processes, advancing webs of material are combined with other advancing webs of material that are typically joined and/or laminated together at one or more so called "marriage" points along the machine direction MD. Similar processes as described above combine sub-assemblies of laminate components to one or more advancing webs of material forming the main chassis of the resulting articles. Webs of material and component parts used to manufacture diapers may include: backsheets, topsheets, absorbent cores, front and/or back ears, fastener components, and various types of elastic webs and components such as leg elastics, barrier leg cuff elastics, waist elastics, and elastic panels (e.g. front/back panels for pants, and back side panels for diapers). Once the desired component parts are assembled, the advancing web(s) and component parts are subjected to a final knife cut to separate the web(s) into discrete absorbent articles (e.g. diapers or pants). The discrete absorbent articles may also then be folded and packaged.

For quality control purposes, absorbent article converting lines may utilize various types of sensor technology to inspect the webs and discrete components added to the webs along the converting line as absorbent articles are constructed. Example sensor technology may include vision systems, photoelectric sensors, proximity sensors, laser or sonic distance detectors, and the like. Product inspection data from the sensors may be communicated to a controller in various ways. In turn, the controller may be programmed to receive product inspection data, and in turn, make adjustments to the manufacturing process. In some instances, the controller may reject defective absorbent articles based on the product inspection data after the final cut at the end of the converting line.

As such, the controller may be programmed with various algorithms adapted to analyze the inspection data and provide desired control functions to the manufacturing process. The complexity and sophistication of the algorithms may vary with the type of inspection data being analyzed. In some configurations, a vision system may be configured to communicate image data from an inspection zone to a controller, wherein aspects of the image data may be analyzed by an algorithm to determine whether control actions should be executed. For example, the image data may be analyzed to determine whether a component is missing or improperly positioned during the assembly process. Depending upon whether other features, such as graphics, bond patterns, or wrinkles, also may be located in the inspection zone, the algorithm may need to be relatively more complex to enable the algorithm to distinguish the component or lack thereof from such other features in the inspection zone. Even more complexity, may be added when substantially simultaneously assessing a plurality of such inspection zones within an entire absorbent article.

Examples of existing quality control systems are described in for example US10,331,113 that relates to systems and methods for manufacturing disposable articles and for detecting and rejecting defective absorbent articles from a converting line by utilising a combination of sensors (including a sensor clock) and virtually segmenting the substrate into a plurality of virtual segments for applying inspection parameters each being assigned a timestamp, ultimately enabling subsequent rejection when certain critical parameters are identified.

Limitations however do exist in human generated inspection algorithms that currently mostly constrain the speed of algorithm development and the scope of what can be inspected. Consequently, it is desirable to generate and utilize algorithms capable of inspections that otherwise cannot be coded by humans to conduct relatively more sophisticated in-process inspection operations of assembled absorbent articles, so called in-process artificial intelligence.

**SUMMARY**

In a first aspect, the disclosure relates to a method for inspecting disposable absorbent articles, the method comprising: providing a controller comprising an inspection algorithm with a convolutional neural network; providing a sensor, preferably selected from the group consisting of an optical sensor, thermal sensor, and combinations thereof; the sensor being in data communication with the controller; providing a reference database, wherein the reference database comprises a plurality of pre-processed images of disposable absorbent articles and/or components thereof that have been labeled with one or more of: a feature or element of the article and/or component thereof; a non-acceptable defect within a feature or element of the article and/or component thereof; an acceptable defect within a feature or element of the article and/or component thereof; an acceptable faultless feature or element of the article and/or component thereof; and combinations thereof; and the method comprising the steps of: training the convolutional neural network through a plurality of iterations or epochs; optionally validating the training, wherein the training step is repeated until a mean average precision score (MAP) of at least 0.9, preferably from 0.91 to 0.96, is attained according to the formula:

$$\mathrm{MAP} = \frac{\sum_{q=1}^{Q} \mathrm{AP}(q)}{Q}$$

where Q is the number of queries; advancing one or more substrates through a converting process along a machine direction to form an array of absorbent articles; creating a plurality of images of said array of absorbent articles with the sensor; transmitting the images from the sensor to the controller; determining at least one of the properties for the labeled component with the inspection algorithm; cutting the substrate into discrete articles; and based on the determination of the least one of the properties for the labeled component, executing a control action, wherein the control action is selected from the group of automatic rejection of one or more articles, automatic machine-setting adjustment, a warning signal for machine maintenance scheduling, and a machine stop command.

In another aspect of the disclosure, that may be further combined with the above first aspect, a method for automatically inspecting one or more components of an absorbent article manufacturing machine or disposable absorbent articles made with said manufacturing machine, the method comprising: providing a controller comprising an inspection algorithm with a convolutional neural network; providing a sensor, wherein said sensor is an acoustic sensor, preferably a microphone; providing a reference database, wherein the reference database comprises a plurality of pre-processed sound recordings of one or more components of said absorbent article manufacturing machine when running or in-operation, preferably said components comprising one or more knives during a cutting step, one or more rotating elements during a conveying step, and/or one or more joining elements, preferably selected from adhesive applicator(s) and sonotrode(s), during a joining or lamination step; or one or more parts of disposable absorbent articles as they are processed by said manufacturing machine; that have been labeled with one or more of: a sound corresponding to a failure or rupture of said one or more components or one or more parts; a sound corresponding to a damage of said one or more components or one or more parts; a sound corresponding to a correct or acceptable running of said one or more components or one or more parts; and the method comprising the steps of: training the convolutional neural network through a plurality of iterations or epochs; optionally validating the training, wherein the training step is repeated until a predefined number of iterations is reached or a mean average precision score (MAP) of at least 0.90, preferably from 0.91 to 0.96, is attained according to the formula:

$$\mathrm{MAP} = \frac{\sum_{q=1}^{Q} \mathrm{AP}(q)}{Q}$$

where Q is the number of queries; advancing one or more substrates through a converting process along a machine direction to form an array of absorbent articles; creating a plurality of sound recordings of said one or more components of the absorbent article manufacturing machine when running or in-operation, or one or more parts of disposable absorbent articles as they are processed by said manufacturing machine, with the sensor; transmitting the sound recordings from the sensor to the controller; determining at least one or more acoustic properties for the labeled component or part with the inspection algorithm, preferably said acoustic property being the sound of said one or more components or parts typically during operation of said machine and/or manufacture of said articles with said machine; cutting the substrate into discrete articles; and based on the determination of the least one of the acoustic properties for the labeled component or part, executing a control action, wherein the control action is selected from the group of automatic rejection of one or more articles, automatic machine-setting adjustment, a warning signal for machine maintenance scheduling, and a machine stop command.

In one aspect, the disclosure relates to a method for inspecting disposable absorbent articles, the method comprising steps of: preprocessing each image of a group of images of articles to label a component of each said article; generating a first inspection algorithm with a neural network based on the preprocessed images, wherein the first inspection algorithm is usable to mask the labeled component in images of articles; providing at least a second inspection algorithm, wherein the second inspection algorithm is usable to determine one or more properties of the labeled component in images of articles, wherein the one or more properties comprise any of a position or location, a size, a shape, orientation and a presence or absence of the component; providing a sensor, preferably selected from the group consisting of an optical sensor, thermal sensor, and combinations thereof; providing a controller, the controller comprising at least the first inspection algorithm; advancing one or more substrates through a converting process along a machine direction to form an array of absorbent articles; creating a plurality of images of said array of absorbent articles with the sensor for processing by the second inspection algorithm; said images comprise at least one of: at least one image taken with reflected light and at least one image taken with transmitted light for each said article in a sequence of articles; at least two images taken with transmitted light from respective at least first and second light sources having different wavelength for each said article in a sequence of articles, wherein the first light source emits electromagnetic waves having a wavelength of 100 to 400 nanometers, preferably from 150 to 350 nanometers, and the second light source electromagnetic waves having a wavelength of 450 to 750 nanometers, preferably from 500 to 700 nanometers; and at least two images taken with transmitted light by a first sensor, typically being an optical sensor, and a second sensor, typically being a thermal sensor, respectively for each said article in a sequence of articles; transmitting the images from the sensor to the controller; determining at least one of the properties for the labeled component with the second inspection algorithm; cutting the substrate into discrete articles; and based on the determination of the least one of the properties for the labeled component, executing a control action, wherein the control action is selected from the group of automatic rejection of one or more articles, automatic machine-setting adjustment, a warning signal for machine maintenance scheduling, and a machine stop command.

## BRIEF DESCRIPTION OF THE FIGURES

**Fig. 1** Illustrates absorbent articles according to embodiments according to the present disclosure.

**Fig. 2** Illustrates absorbent articles comprising a defect according to embodiments according to the present disclosure.

**Fig. 3** Is a schematic representation of a system according to an embodiment of the present disclosure.

## DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements, such as a laminate.

"Laminate" refers to elements being attached together in a layered arrangement.

"Thickness or caliper" herein are used interchangeably, and refer to the caliper typically measured as follows:

at 0.5 kPa and at least five measurements are averaged. A typical testing device is a Thwing Albert ProGage system. The diameter of the foot is between 50 mm to 60 mm. The dwell time is 2 seconds for each measurement. The sample is to be stored at 23 + 2°C and at 50 + 2% relative humidity for 24 hours with no compression, then subjected to the fabric thickness measurement. The preference is to make measurements on the base substrate before modification, however, if this material is not available an alternative method can be used. For a structured substrate, the thickness of the first regions in between the second regions (displaced fiber regions) can be determined by using a electronic thickness gauge (for instance available from McMaster-Carr catalog as Mitutoyo No 547- 500). These electronic thickness gauges can have the tips changed to measure very small areas. For example, a blade shaped tip can be used that is 6.6mm long and 1 mm wide. Flat round tips can also be inserted that measure area down below 1.5mm in diameter. For measuring on the structured substrate, these tips are to be inserted between the structured regions to measure the as-produced fabric thickness. The pressure used in the measurement technique cannot be carefully controlled using this technique, with the applied pressure being generally higher than 0.5kPa.

The term "topsheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

The term "backsheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

As used herein, the term "transverse" or "lateral" refers to a line, axis, or direction which lies within the plane of the absorbent article and is generally perpendicular to the longitudinal direction.

As used herein, the term "cellulosic" or "cellulose" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 $\mu$m, preferably between 300 to 600 $\mu$m, more preferably between 400 to 500 $\mu$m. Superabsorbent materials suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present disclosure in an amount up to about 90% by weight.

By "substantially", it is meant at least the majority of the structure referred to.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

As used herein, the "body-facing" or "bodyside" or "skin-facing" surface means that surface of the article or component which is intended to be disposed toward or placed adjacent to the body (e.g. the face) of the wearer during ordinary use, while the "outward", "outward-facing" surface is on the opposite side, and is intended to be disposed to face away from the wearer's body during ordinary use.

The term "convolutional neural network ("CNN", or "ConvNet")" is a class of deep neural networks, most commonly applied to analyzing visual imagery. They are sometimes also referred to as shift invariant or space invariant artificial neural networks (SIANN), based on the shared-weight architecture of the convolution kernels that shift over input features and provide translation equivariant responses. Convolutional networks are generally inspired by biological processes in that the connectivity pattern between neurons resembles the organization of the animal visual cortex. Individual cortical neurons respond to stimuli only in a restricted region of the visual field known as the receptive field. The receptive fields of different neurons partially overlap such that they cover the entire visual field. CNNs typically use relatively little pre-processing compared to other image classification algorithms. This means that the network learns to optimize the filters (or kernels) through automated learning, whereas in traditional algorithms these filters are hand-engineered. The "convolutional" network generally employs a mathematical operation called convolution. A convolutional neural network typically comprises an input layer, hidden layers and an output layer. In any feed-forward neural network, any middle layers are called hidden because their inputs and outputs are masked by the activation function and final convolution. In a convolutional neural network, the hidden layers include layers that perform convolutions. Typically this includes a layer that does multiplication or other "dot product" (i.e. an algebraic operation that takes two equal-length sequences of numbers (usually coordinate vectors), and returns a single number), and its activation function is commonly "ReLU" (i.e. an activation function defined as the positive part of its argument: $f(x) = x^+ = \max(0,x)$ where x is the input to a neuron. This is also known as a ramp function and is generally analogous to half-wave rectification in electrical engineering). This is followed by other layers such as pooling layers, fully connected layers, and normalization layers.

The term "pipeline(s)" or "machine learning pipeline" generally refers to a way to codify and automate the workflow it takes to produce a machine learning model. Machine learning pipelines consist of multiple sequential steps that do everything from data extraction and preprocessing to model training and deployment. Typically it enables a sequence of data to be transformed and correlated together in a model that can be tested and evaluated to achieve an outcome.

The term "epoch" generally is a hyperparameter that defines the number times that the learning algorithm will work through the entire training dataset. One epoch means that each sample in the training dataset has had an opportunity to update the internal model parameters. An epoch is comprised of one or more batches.

Embodiments of the articles and processes according to the disclosure will now be described. It is understood that technical features described in one or more embodiments may be combined with one or more other embodiments without departing from the intention of the disclosure and without generalization therefrom.

THE SYSTEM AND PROCESS

The present disclosure relates to systems and processes for manufacturing absorbent articles.

In an aspect, the disclosure relates to a method for inspecting disposable absorbent articles, the method comprising: providing a controller comprising an inspection algorithm with a convolutional neural network; providing a sensor, preferably selected from the group consisting of an optical sensor, thermal sensor, and combinations thereof; the sensor being in data communication with the controller; providing a reference database, wherein the reference database comprises a plurality of pre-processed images of disposable absorbent articles and/or components thereof that have been labeled with one or more of: a feature or element of the article and/or component thereof; a non-acceptable defect within a feature or element of the article and/or component thereof; an acceptable defect within a feature or element of the article and/or component thereof; an acceptable faultless feature or element of the article and/or component thereof; and combinations thereof; and the method comprising the steps of: training the convolutional neural network through a plurality of iterations or epochs; optionally validating the training, wherein the training step is repeated until a mean average precision score (MAP) of at least 0.9, preferably from 0.91 to 0.96, is attained according to the formula:

$$MAP = \frac{\sum_{q=1}^{Q} AP(q)}{Q}$$

where Q is the number of queries; advancing one or more substrates through a converting process along a machine direction to form an array of absorbent articles; creating a plurality of images of said array of absorbent articles with the sensor; transmitting the images from the sensor to the controller; determining at least one of the properties for the labeled component with the inspection algorithm; cutting the substrate into discrete articles; and based on the determination of the least one of the properties for the labeled component, executing a control action, wherein the control action is selected from the group of automatic rejection of one or more articles, automatic machine-setting adjustment, a warning signal for machine maintenance scheduling, and a machine stop command.

In another aspect of the disclosure, that may be further combined with the above first aspect, a method for automatically inspecting one or more components of a diaper manufacturing machine or disposable absorbent articles made with said manufacturing machine, the method comprising: providing a controller comprising an inspection algorithm with a convolutional neural network; providing a sensor, wherein said sensor is an acoustic sensor, preferably a microphone; providing a reference database, wherein the reference database comprises a plurality of pre-processed sound recordings of one or more components of a diaper manufacturing machine when running or in-operation, preferably said components comprising one or more knives during a cutting step, one or more rotating elements during a conveying step, and/or one or more joining elements, preferably selected from adhesive applicator(s) and sonotrode(s), during a joining or lamination step; or one or more parts of disposable absorbent articles as they are processed by said manufacturing machine; that have been labeled with one or more of: a sound corresponding to a failure or rupture of said one or more components or one or more parts; a sound corresponding to a damage of said one or more components or one or more parts; a sound corresponding to a correct or acceptable running of said one or more components or one or more parts; and the method comprising the steps of: training the convolutional neural network through a plurality of iterations or epochs; optionally validating the training, wherein the training step is repeated until a predefined number of iterations is reached or a mean average precision score (MAP) of at least 0.90, preferably from 0.91 to 0.96, is attained according to the formula:

$$MAP = \frac{\sum_{q=1}^{Q} AP(q)}{Q}$$

where Q is the number of queries;
advancing one or more substrates through a converting process along a machine direction to form an array of absorbent articles; creating a plurality of sound recordings of said one or more components of a diaper manufacturing machine when running or in-operation, or one or more parts of disposable absorbent articles as they are processed by said manufacturing machine, with the sensor; transmitting the sound recordings from the sensor to the controller; determining at least one of the properties for the labeled component or part with the inspection algorithm; cutting the substrate into discrete articles; and based on the determination of the least one of the properties for the labeled component or part, executing a control action, wherein the control action is selected from the group of automatic rejection of one or more articles, automatic machine-setting adjustment, a warning signal for machine maintenance scheduling, and a machine stop command. Advantageously this allows for predictive failure monitoring of machine components like knives cutting or adhesive applicators/sonotrodes laminating etc. in operation since there are distinctive sounds that are made when functioning correctly vs when cracks or other failure modes start to occur, and this allows for a further layer of automatic detection and inspection which is not possible to the same frequency with human operators/maintenance teams and allows for early intervention rather than waiting for catastrophic failures to occur.

Generally speaking and referring to the above mentioned formulas, Average Precision (AP) is a metric that can be used to measure the accuracy of the output of the neural network. Precision and recall are single-value metrics based on the whole list of data files returned by the system. Average Precision is defined as the integral of precision p(r) over all recall values from r = 0 to r = 1:

$$AP = \int_0^1 p(r)dr$$

That is the area under the precision-recall curve. For practical computations, this integral is replaced with a finite sum over every position in the ranked sequence of data files:

$$AP = \sum_{k=1}^{n} P(k)\Delta r(k)$$

where $k$ is the rank in the sequence of retrieved data files, $n$ is the number of retrieved data files, $P(k)$ is the precision at cut-off $k$ in the list, and $\Delta r(k)$ is the change in recall from items $k$ - 1 to $k$. Advantageously, including such validation step that pauses the training once the mentioned MAP range is reached allows to optimize time and energy consumption of the training step whilst achieving sufficient accuracy required for processing of pre-processed images for inspection of specifically disposable absorbent articles.

In an embodiment, a confusion matrix may be used to allow visualisation of the performance of the inspection algorithm in a tabular layout. Typically each row of the matrix represents the instances in a predicted class, while each column represents the instances in an actual class (or vice versa). The name stems from the fact that it makes it easy to see whether the system is confusing two (or more) classes (i.e. mis-labelling one as another). Such a confusion matrix may be adopted during a supervised training (or concept learning) phase within the training step. Typically such phase comprises the step of tagging data by a human operator (e.g. inputting data tags to e.g. identify a component of the absorbent article, like an elastic side panel, or channel in a core etc.; identify a defect or feature in the component, and so on).

In a very simplified example of the above embodiment, this could take the form of images classified as "good" or "bad" with "1"s and "0"s respectively (i.e. 1=good; and 0=bad) or vice versa. In an example, a given sample of images may comprise 12 images 8 of which are "good" and 4 are "bad" and can be represented mathematically as:

Actual = [1,1,1,1,1,1,1,1,0,0,0,0]

A classifier within the algorithm may then be trained to distinguish between "good" and "bad"; the 12 pictures are taken and run through the classifier, in this example we assume the classifier makes 9 accurate predictions and misses 3 e.g. 2 wrongly predicted "good" (first two predictions in this example, see the "0" below in the first positions) and 1 wrongly predicted "bad" (last prediction in this example, see the "1" in the last position):

Prediction = [0,0,1,1,1,1,1,1,0,0,0,1]

With such two labelled sets (actual and predictions) a confusion matrix can be generated that will summarize the results of testing the classifier:

| Actual / Predicted | Good | Bad |
|---|---|---|
| Good | **6** | 1 |
| Bad | 2 | **3** |

In this confusion matrix, of the 8 "good" pictures, the algorithm judged that 2 were "bad", and of the 4 "bad" pictures, it predicted that 1 were "good". All correct predictions are located in the diagonal of the table (highlighted in **bold**), so it is easy to visually inspect the table for prediction errors, as they will be represented by values outside the diagonal. The final table of confusion would contain the average values for all classes combined.

In terms of sensitivity and specificity, the confusion matrix may therefore be represented as follows:

| Actual / Predicted | P | N |
|---|---|---|
| P | **TP** | FP |
| N | FN | **TN** |

Where P is the condition positive (i.e. number of real positives in a data set e.g. within the total number of images to be inspected), N is the condition negative (i.e. number of real negative cases in the data set), TP is the true positive (correct acceptance), TN is the true negative (correct rejection), FP is the false positive (false alarm, type I error or underestimation), and FN is the false negative (miss, type II error or overestimation).

In an embodiment, a matching matrix may be used in a learning phase of the training step to enable the inspection algorithm to learn patterns from untagged data, wherein the inspection algorithm exhibits self-organization that captures patterns as neuronal predilections or probability densities without the need for a step of tagging data by a human operator within the learning phase.

In an embodiment, a combination of the previously two described embodiments may be used typically wherein a smaller portion of the data is tagged in a so called semi-supervised learning phase of the training step. Advantageously, this provides for an optimal training of the algorithm that balances minimal use of human operator time with reduced computing power, energy and time used by the algorithm in self-organization.

Preferably, the images comprise at least one of: at least one image taken with reflected light and at least one image taken with transmitted light for each said article in a sequence of articles; at least two images taken with transmitted light from respective at least first and second light sources having different wavelength for each said article in a sequence of articles, wherein the first light source emits electromagnetic waves having a wavelength of 100 to 400 nanometers, preferably from 150 to 350 nanometers, and the second light source electromagnetic waves having a wavelength of 450 to 750 nanometers, preferably from 500 to 700 nanometers; and at least two images taken with transmitted light by a first sensor, typically being an optical sensor, and a second sensor, typically being a thermal sensor, respectively for each said article in a sequence of articles. Advantageously this allows for accurate detection of indicia e.g. defects at multiple locations of the article including through the thickness thereof (as well as its outer surface(s)), as well as automatic detection of glue application areas for automatic inspection thereof.

In an embodiment, the pre-processed images are automatically labeled by: said inspection algorithm after the training step or by a further inspection algorithm. This allows to advantageously automatically continue building the dataset of labeled images to significantly increase the reference database content for further automatic learning and accuracy of detection by the inspection software and hardware.

Preferably, the method further comprises the step of automatically feeding at least a portion of captured images to the reference database to increase the number of pre-processed images for the inspection algorithm to use in the determination step, preferably wherein said at least portion comprises images that are different from existing images in the reference database prior to the feeding step. Advantageously this aids memory allocation and size optimization within the reference database to e.g. ensure high speed of operation at all times even when the reference database is stored in the Cloud.

In an embodiment, the inspection algorithm is trained until a predefined validation loss is attained. At the most basic level, a loss function quantifies how "good" or "bad" a given predictor is at classifying the input data points in a dataset, the smaller the loss, the better a job the classifier is at modeling the relationship between the input data and the output targets.

In an embodiment, the inspection algorithm automatically stops if the validation loss does not improve over 10 epochs or less, preferably from 5 to 9 epochs. Preferably, the convolutional neural network of the inspection algorithm uses a validation set on every epoch, because training too long can cause over-fitting, and models don't recover from that, they just get worse form that point, this function aims at stopping the training when it has not improved for a long while, or starts to get worse.

Examples of suitable software that can be implemented in image machine learning/inspection algorithms herein may be selected from: TensorFlow (this is an open-source toolkit that can be used to build machine learning pipelines that can build scalable systems to process data. It provides support and functions for various applications of ML such as Computer Vision, NLP and Reinforcement Learning); Keras (is an open-source neural network library that provides support for Python. It provides support for the implementation of Convolutional Neural Networks, Recurrent Neural Networks as well as both. It is capable of running seamlessly on the CPU and GPU; OpenCV (is a tool that has C++, C, Python and Java interfaces and supports Windows, Linux, Mac OS, iOS and Android for computational efficiency and with a strong focus on real-time applications, written in optimized C/C++, the library can take advantage of multi-core processing and enabled to take advantage of the hardware acceleration of the underlying heterogeneous compute platform); SimpleCV (is an open source framework for building computer vision applications, user can get access to several high-powered computer vision libraries such as OpenCV without having to learn about bit depths, file formats, color spaces, buffer management, eigenvalues, or matrix versus bitmap storage); Torch (Scientific computing framework with wide support for machine learning algorithms that puts GPUs first. Easy to use and efficient. Fast scripting, LuaJIT, and underlying C/CUDA implementation); Shogun (an open-source machine learning software. It is also written in C++. It supports various languages like Python, R, Scala, C#, Ruby etc.); and Oryx 2 (makes use of Lambda Architecture for real-time and large-scale machine learning processing. This model was built on top of the Apache Spark architecture that involves packaged functions for building rapid-prototyping and applications. It facilitates end to end model development for collaborative filtering, classification, regression as well as clustering operations).

In an embodiment the training step comprises a freeze backbone step which comprises the step of freezing one or more layers of the neural network so that the weights of said one ore more layers can no longer be modified once a predetermined mean average precision score (MAP) is reached. Advantageously the introduction of such a step permits to limit "over-training" of the algorithm that would otherwise have higher risks to suffer from time lapses or excessive processing times without adding to precision of the logic decision making attributes.

In an embodiment, the pre-processed images are tagged with metadata comprising information about at least one of: the image source type such as whether reflected or transmitted light was used or whether other non-visual imaging sources e.g. heat were used; absorbent article type such as a diaper or pant; element or component of an absorbent article such as an elastic ear panel or absorbent core; a command prompt indicator such as qualifiers selected from acceptable or not acceptable; and combinations thereof; and preferably wherein the reference database is arranged to cluster said images based on said metadata; and preferably wherein the inspection algorithm automatically compares one or more captured images, taken in the step of creating a plurality of images of said array of absorbent articles with the sensor, with the most similar images in the reference database based on said metadata. Advantageously this allows for effective and quick comparison of acquired images with the most relevant reference image(s) from the reference database for the correct decision to be taken by the logic in the shortest possible time frame.

In an embodiment, the training step comprises the step of creating a pipeline that is further transmitted to the, preferably trained, inspection algorithm or further inspection algorithm for pre-processing prior to being transmitted to the reference database. Advantageously, this helps to optimize automatic further training of the neural network logic.

In an embodiment, the method comprises the steps of: preprocessing each image of a group of images of articles to label a component of each said article; generating a first inspection algorithm with a neural network based on the preprocessed images, wherein the first inspection algorithm is usable to mask the labeled component in images of articles; providing at least a second inspection algorithm, wherein the second inspection algorithm is usable to determine one or more properties of the labeled component in images of articles, wherein the one or more properties comprise any of a position or location, a size, a shape, orientation and a presence or absence of the component; providing a sensor, preferably selected from the group consisting of an optical sensor, thermal sensor, and combinations thereof; providing a controller, the controller comprising at least the first inspection algorithm; advancing one or more substrates through a converting process along a machine direction to form an array of absorbent articles; creating a plurality of images of said array of absorbent articles with the sensor for processing by the second inspection algorithm; said images comprise at least one of: at least one image taken with reflected light and at least one image taken with transmitted light for each said article in a sequence of articles; at least two images taken with transmitted light from respective at least first and second light sources having different wavelength for each said article in a sequence of articles, wherein the first light source emits electromagnetic waves having a wavelength of 100 to 400 nanometers, preferably from 150 to 350 nanometers, and the second light source electromagnetic waves having a wavelength of 450 to 750 nanometers, preferably from 500 to 700 nanometers (the first light source may be positioned downstream the second light source in a machine direction MD or vice versa); and at least two images taken with transmitted light by a first sensor, typically being an optical sensor, and a second sensor, typically being a thermal sensor, respectively for each said article in a sequence of articles (the first light sensor may be positioned downstream the second light sensor in a machine direction MD or vice versa); transmitting the images from the sensor to the controller; determining at least one of the properties for the labeled component with the second inspection algorithm; cutting the substrate into discrete articles; and based on the determination of the least one of the properties for the labeled component, executing a control action, wherein the control action is selected from the group of automatic rejection of one or more articles, automatic machine-setting adjustment, a warning signal for machine maintenance scheduling, and a machine stop command.

Preferably, the first light source emits a UV light and the second light source emits visible light. Typically the second light source is arranged to transmit light through the absorbent article and is typically arranged at an opposite position to the sensor, typically an optical sensor, such that the absorbent article at an imaging position is interposed between the light source and the sensor such that light is transmitted through the article substantially concurrently to an image being taken. The first light source is typically arranged to reflect light over the absorbent article and is typically arranged at a position proximal to the sensor, typically the optical sensor, such that the sensor and the light source are generally both positioned above a surface of the absorbent article. Such arrangement allows for more accurate detection of defects relating to a combination of components such as glue and core positioning, glue and fold or holes presence etc as well as further advantages that will become apparent herein below.

Preferably the preprocessed image is digitally tagged with metadata corresponding to the imaging source (e.g. to distinguish an optical image by transmitted light, reflected light, or UV, or thermal image etc.) so that the first and/or second inspection algorithm can directly compare images from the same imaging source within a reference database as will be explained in more detail hereinbelow. Preferably the reference database comprises data comprising information related to collected images including metadata relating to the imaging source and typically images are clustered within said database according to said metadata.

In an embodiment, the method comprises the steps of: preprocessing each image of a group of images of articles to label a component of each said article; generating a first inspection algorithm with a, preferably convolutional, neural network based on the preprocessed images, wherein the first inspection algorithm is usable to mask (typically for hiding the irrelevant elements/components or more preferably enhancing/highlighting the relevant elements/components to be inspected) the labeled component in images of articles typically to generate processed images; providing a second inspection algorithm (preferably with a convolutional neural network typically based on processed images), wherein the second inspection algorithm is usable to determine one or more properties of the labeled component in images of articles, wherein the one or more properties comprise any of a position or location, a size, a shape, orientation and a presence of the component; providing a sensor, preferably an optical sensor; providing a controller, the controller comprising the first inspection algorithm; advancing one or more substrates through a converting process along a machine direction to form an array of absorbent articles; creating a plurality of images of said array of absorbent articles with the sensor preferably for processing by the second inspection algorithm; said images comprising at least one image taken with reflected light and at least one image taken with transmitted light for each said article in a sequence of articles; communicating or transmitting the images from the sensor to the controller; determining at least one of the properties for the labeled component with the second inspection algorithm; cutting the substrate into discrete articles; and based on the determination of the least one of the properties for the labeled component, executing a control action, wherein the control action is selected from the group of automatic rejection of one or more articles, automatic machine-setting adjustment, a warning signal for machine maintenance scheduling, and a machine stop command.

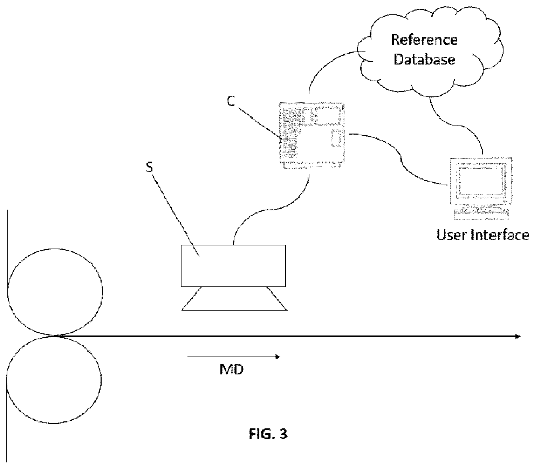

Systems herein may comprise a sensor, preferably an optical sensor and/or thermal sensor; and a controller, the controller comprising the first inspection algorithm; and a graphics processing unit (GPU) comprising the second inspection algorithm. For illustration purposes, Fig. 3 shows a non-limiting schematic representation of an exemplary embodiment wherein the system comprises an optical sensor (S), typically in the form a camera inspection subsystem as described hereinbelow, a controller (C); a reference database exemplarily remotely stored within the Cloud; and a user interface that may comprise a graphics processing unit (GPU) therein. The reference database may be in data communication with the controller (C) and/or user interface, and the sensor (S) may be in data communication with the controller (C). The controller may futher comprise one or more processing units (for example a central processing unit, CPU) and an optional memory. In this example as a laminate sub-assembly travels along the machine direction MD, the sensor (S) takes sequential pictures and the system runs an inspection process as described in more detail hereinbelow.

In a highly preferred embodiment, the one or more properties comprise any of: the absence or presence of discontinuities within a channel 7 free of absorbent material within the absorbent core of said articles; positioning of a fold of one or more core wrap layers within the absorbent core or absorbent assembly; presence or absence of a fold within an elastic side panel 3,4 said fold positioned between a topsheet 8 and a backsheet 9 of the absorbent article wherein said topsheet and backsheet sandwich the absorbent core or absorbent assembly therebetween; positioning of an acquisition distribution layer with respect to a perimeter of an absorbent core or absorbent assembly of the article. Advantageously image analysis of images taken with both reflected light and transmitted light allows for accurate intra-layer inspection of components that enable detection of more complex hidden product defects that may not only impact usability and aesthetics of the articles but further product performance attributes.

The systems (sometimes herein referred to as AI-systems) herein may comprise one or more camera sub-systems; one or more graphics processing units (GPU); and deep learning algorithm or convolutional neural network.

As a first step, the software may be generally trained with two sets of pre-recorded and pre-evaluated camera images: one including only "good" products, the other one including only "bad" products. In that way, the neural network learns to distinguish between the two categories. Some examples of bad products are: missing components, misplaced components, folds, etc. A "reference" database is generated by collecting said sets of pre-recorded and pre-evaluated camera images. It is advantageous to continually build the database with new said sets as the process is running to improve and evolve the accuracy of the system with time, therefore it is preferred that the database is stored within the cloud and accessable by the later described processing means.

After being trained, the software (or algorithm) is typically used to evaluate product images of a running line. Good products can pass, whereas bad products are rejected by automatic triggers signalled by the system to the process line to deviate respective products along a rejects path that is different from the path that good products follow downstream towards the folding and packaging units of the production line. For that purpose, the inspection system or deep learning software may be connected with the machine control system (SPS).

The number of pre-recorded images used for the training of the neural network is preferably greater than 1,000; preferably greater than 5,000; more preferably greater than 10,000; even more preferably greater than 15,000. Advantageously this allows for a significantly robust training period of the neural network that ensures accuracy of the system.

In an embodiment, a combination or convolution of different parameters as input for decision taking may be used (in contrast to existing systems which trigger a product reject based on a list of separate parameters).

In a preferred embodiment, the camera sub-systems used each comprise one or more high resolution 2k cameras. Advantageously this allows for more fine pixel allocation in the inspection analysis of rendered images.

In an embodiment, the collected data from ongoing production is used for automatically changing machine settings. Advantageously this allows for further finetuning to an accuracy degree significantly greater than human operated actions.

In an embodiment, a combination of different inputs is used, e.g. two or more camera images, or one camera image and additional sensor signals.

In an embodiment, the output of the AI-system may be combined with the output(s) of other system(s), e.g. sensors, to take a final decision on rejecting product or not.

The system herein may use a combination of different types of lighting such as transmitted and/or reflected. Typically, data points for images captured with each of the lighting conditions are combined to provide an overall indication of the absorbent article condition.

The system herein may use variable ranges of wavelengths for the light source, including non-visible light (e.g. UV). This is particularly beneficial when automatically inspecting for example the correctness of adhesive application.

The system herein may comprise detectors and imaging techniques such as infrared or terahertz waves.

What makes absorbent disposables different to many other types of products (that are already processed with AI based vision systems) is that the different components are translucent (to different degrees).

Advantageously, when analysing aborbent articles and sub-assemblies thereof that are translucent (albeit often to varying extents e.g. nonwoven substrates having higher translucency vs films), particularly if transmitted (shine through) light is used, the camera images will include overlays of different layers of materials, each of the layers contributing different levels of light attenuation. Each of these can be advantageously inspected to provide multi-component analysis that may or may not be combined with further surface information and/or data gathered from images collected via reflected light.

In a general form, the camera system comprises a transmitter of electromagnetic waves (preferably: visible light) together with a detector (preferably: a camera). The detected array of data points (preferably: an image) is processed via a neural network which has been trained with pre-labeled sets of "good" (to keep) and "bad" (to reject) example images during a learning phase. For each of the images processed, the neural network typically decides whether that image falls into the good or bad category. That decision is then typically transmitted to the main control system of the production line, triggering the corresponding product to be either kept or rejected (e.g. exhausted/scrapped).

In an embodiment, the camera images are only processed but not stored; or the camera images are first stored, then processed further; or preferably the camera images are first processed, then stored. The latter has the advantage of allowing faster processing whilst still retaining the images that can be added to the reference database thus growing the "knowledge" and accuracy of the AI-system.

As an example, purely for non-limiting illustration purpose, after going through the process above, individual samples can be deliberately taken out of the line, inspected by an experienced person and classified as being acceptable or or not, typically creating a pipeline. That expert verdict can be compared with the neural network decision, and further be manually integrated within the reference database so as to further build accuracy as the system is used. In a similar manner, the recorded camera image (rather than the physical product sample) can be inspected by an experienced person, and again this expert verdict be compared with the neural network decision.

In an example, on the current baby diaper lines, various machine settings are typically adjusted manually, e.g. by turning a spindle or otherwise-like mechanical manipulations. This has several disadvantages, the biggest one being that it is extremely challenging to reproduce all such machine settings correctly and quickly, e.g. for adjusting the line to a different type of raw material or a different product size.

To overcome such issues, such systems comprising convolution neural networks may further be used to directly feedback to machine elements that are normally adjusted by manual operation. The system may be capable to know the position of e.g. any gluehead, even if the system is turned off or moved and/or displaced by hand, for example due to the potentiometer being connected to the main axis. Such systems may comprise: a server (e.g. Raspberry Pi); a microcontroller (e.g. ATMEGA328P); a motor (e.g. NEMA17); a 10 turn potentiometer; an RF wireless system (e.g. NRF24); and a Step down Voltage regulator. As such system can collect the data from each position, wirelessly, automatic adjustment at all positions of e.g. glueheads (but generally any production line component) optionally just by a user pre-selecting the product to be run on the production line from an input screen of a user interface.

The control system of the manufacturing line can be advantageously programmed to adjust certain machine settings autonomously, without the need for an operator to interfere (as is the case in production lines today). For example, if the outside edge of the rear side panel is detected to be placed too far away from the chassis, the control system can activate an actuator so that the rear side panels of the following products are driven back into an acceptable position in response to an automated signal generated from the AI-system.

In one embodiment, the process runs predictively meaning that the control systems recognizes a trend in the position of a certain product component, e.g. if the rear side panels are moving more and more away from the chassis from one product to the next, over a series of consecutive products. Before those ears reach an unacceptable position (which would require the products to be rejected and scrapped), the control system interferes so as to adjust the ear position via an actuator. As a result, the rear side panel position is kept under control, without any scrap product generated, and without intervention of an operator required.

In general, the self-adjusting process may comprise: a vision system, and actuators to change machine settings. For the vision system, it is not mandatory but highly prefered to be deep learning based AI-system. In particular, the predictive adjustment process would greatly benefit from the application of neural network algorithms.

Regarding the energy consumption and predictive maintenance of production lines, talking about lateral channel blowers and big low voltage motors, close loop system capable to measure airflow, deltapressure in filters; can all be optimised with incorporation of an AI-system. A predictive maintenance and/or energy optimising system may comprise: an airflow thermocouple sensor; a vacuum sensor (e.g. FESTO); a Microcontroller (e.g. ATMEGA328P); an RF wireless module (e.g. NRF24); and a DataCloud.

Being capable of measuring the airflow and pressure in the line, allows to define which are the right values for a smooth process and reducing the troubleshoot when issues happend. Meaning that being able to monitorize all such parameters allows to input in the frequency converters and booster the right RPM ensuring the correct airflow/pressure at the least consumption of energy, as well as automatically detecting when the filter is stuck and a maintenance is needed. Reducing the vacuum until the optimal value will ensure that the least operating suction power is used that further allows to minimise dirt and/or contamination.

The systems and processes herein may be configured to inspect substrates and components on a production line (herein also referred to as a "converting apparatus") utilizing an inspection algorithm generated with a neural network, preferably a convolutional neural network. During the manufacture of absorbent articles, a substrate may be advanced through a converting process while combining with other substrates and/or adding component parts to the substrate. In turn, the substrate with component parts added thereto may be cut into discrete absorbent articles. As such, an inspection system may be configured to inspect the substrate and/or component parts during the assembly process. As discussed in more detail below, the inspection system may include a controller and a sensor. The controller includes an inspection algorithm generated with a convolutional neural network based on convolutional neural network parameters. The inspection algorithm may be created based in part from a group of images of absorbent articles. Each of the images may be preprocessed to label a component of the absorbent article. The component may be, for instance, a landing zone, an ear, a tab, among a variety of other features or attributes, including contaminants such as a foreign material, grease, or dirt, or other undesirable transformation of the absorbent article such as a tear, a wrinkle, a fold, and so forth. In addition to labeling a component, preprocessing the image can optionally include cropping the image, scaling the image, resizing the image, and so forth. The preprocessed images may then be provided to a training algorithm to create the convolutional neural network parameters used to generate the inspection algorithm. During inspection operations, the sensor may be adapted to create inspection data of at least one of the substrate and component parts, and then communicate the inspection data to the controller. The labeled components of the substrate and component parts from the training set may then be identified by analyzing the inspection data with the inspection algorithm. Subsequent to identifying the labeled component, a second inspection algorithm may then be utilized to identify one or more properties of the labeled component. In turn, the controller may execute a control action based on properties of the identified component.

It is to be appreciated that the systems and methods disclosed herein are applicable to work with various types of converting processes and/or machines, such as for example, absorbent article manufacturing, and/or packaging processes. For illustration purposes, the methods and apparatuses are discussed below in the context of manufacturing diapers, although this disclosure is not so limited. The methods and apparatuses are applicable to converting processes for feminine hygiene products, for example.

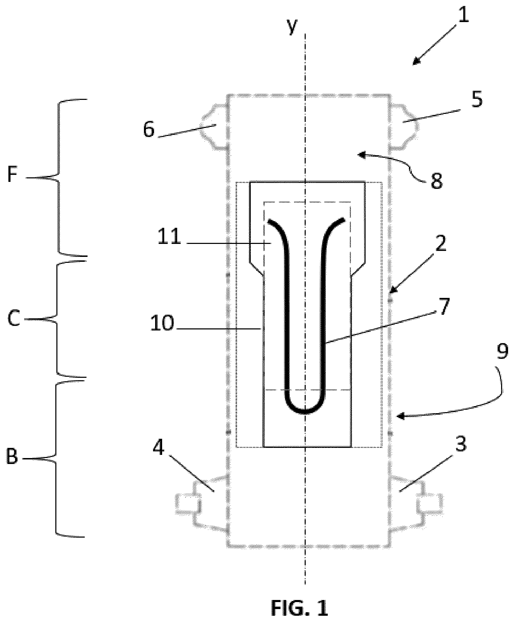

For the purposes of a specific illustration, FIG. 1 show an example of an absorbent article 1 that may be assembled in accordance with the methods and apparatuses disclosed herein. In particular, FIG. 1 shows one example of a plan view of an absorbent article 1 configured as a taped diaper, with the portion of the diaper that faces towards a wearer oriented towards the viewer. The taped diaper shown in FIG. 1 includes a chassis 2, first and second rear side panels 3 and 4; first and second front side panels 5 and 6; and a channel 7 typically formed by bonding (for example by use of adhesive) top and bottom core wrap layer(s) of an absorbent core that are generally used to sandwich absorbent material therein, and such that the channel 7 is substantially free of absorbent material. Typically the absorbent material comprises superabsorbent polymer particles and/or cellulose fibers (also sometimes referred to as airfelt).

The rear side panels 3 and 4 are preferably elastic. In such embodiments, the panels typically consist of a laminate structure comprising at least one nonwoven substrate and an elastic film adhered thereto. Generally such panels comprise at least two nonwoven substrates that sandwich the elastic film therebetween.

As shown in FIG. 1, the diaper 1 and the chassis 2 each include a front waist region F, a back waist region B, and a crotch region C disposed intermediate the front and back waist regions. In some configurations, the length of each of the front waist region, back waist region, and crotch region may be ⅓ of the length of the absorbent article 1. The absorbent article typically also includes a perimeter formed by respective front and back transversal edges and oppositely disposed lateral longitudinal edges (typically extending substantially parallel to a longitudinal axis y that further generally corresponds to the machine direction in processes of production described herein) connecting the front and back transverse edges.

Absorbent articles herein include an inner, body facing surface, and an outer, garment facing surface. The chassis 2 may include a topsheet 8 and a backsheet 9. The chassis may also include an absorbent assembly, including an absorbent core 10, disposed between a portion of the topsheet 8 and the backsheet 9. The absorbent articles herein may also include other features, such as leg elastics and/or leg cuffs, an elastic waist region, and/or flaps, e.g., side panels and/or ears, to enhance the fits around the legs and waist of the wearer.

With continued reference to FIG. 1, each side panel 3,4,5,6 and/or fastening member may form a portion of or may be permanently bonded, adhered or otherwise joined directly or indirectly to the chassis 2, for example in one of the front waist region or the back waist region. Alternatively, the fastening members may form a portion of or may be permanently bonded, adhered or otherwise joined directly or indirectly to the first and second rear panels 3,4 at or adjacent the distal edge of the panel and/or the first and second front side panels 5 and 6 at or adjacent the distal edge of the side panel. It is to be appreciated that the fastening members and/or side panels may be assembled in various ways as known in the art.

Asorbent articles herein may further comprise an acquisition distribution layer (ADL) 11 typically positioned between the topsheet 8 and the absorbent core (typically the top/upper core wrap layer thereof) and generally having the function of improving fluid distribution to the absorbent core.

In a preferred embodiment, at least 90%, preferably all components of the absorbent article are translucent. It is appreciated and preferable that one or more such components may have different levels of translucency for example nonwoven layers such as the topsheet or ADL will have a higher translucency compared to films such as the backsheet and/or elastic films such as used within elastic side panels/ears.

Absorbent article converting processes suitable for use herein include a converting line or production machine configured to manufacture absorbent articles. It is to be appreciated that the systems and methods disclosed herein are applicable to work with various types of converting processes and/or machines. The converting line may include one or more motors that drive transport systems, such as a nip roll, to move article (e.g. diaper) substrates and component materials through the manufacturing process. For example, a base substrate and two or more auxiliary substrates and/or components of material used to construct portions of the diapers. The substrates may be provided as rolls and fed into the converting line. It is to be appreciated that material of the auxiliary substrates may be supplied in various ways. For example, a first auxiliary substrate in the form of a continuous substrate, and a second auxiliary substrate in the form of individual components. It is to be appreciated that the auxiliary substrates may be transferred to the base substrate through various types of transfer mechanisms. For example, the individual components may be in the form of side panels such as shown in FIG. 1. As such, the side panels may be transferred to the base substrate via a transfer mechanism in the form of a servo patch placer mechanism with methods known in the art. In addition, the nip roll may be configured create bonds between the side panels and the chassis. For example, the nip roll may be configured as a mechanical bonding unit (or as an adhesive applicator positioned upstream of a nip or pressure roller). In another example, the nip roll may be configured as a thermal bonding. It is also to be appreciated that the various substrates can be used to construct various components of the absorbent articles, such as backsheets, topsheets, ears, leg cuffs, elastic waist features, and absorbent cores.

As the base substrate advances through the converting line, the base substrate is combined with the auxiliary substrates and/or discrete components to create a continuous length of absorbent articles. At a downstream portion of the converting process, the continuous length of absorbent articles is subjected to a final knife and cut to create separate and discrete absorbent articles in the form of diapers. Defective articles may be subject to a rejection system and removed from the process. For example, defective articles may be channeled to a reject bin. It is to be appreciated that the term "reject bin" is used herein generically to designate the location where rejected diapers may be conveyed. As such, the reject bin may include various rejection systems/locations even remote from the production machine. For example, the reject bin may include additional systems such as conveyors and/or pneumatic systems to provide additional transport or conveyance of rejected diapers to other locations. Articles that are not deemed to be defective may be subject to further processing steps, such as folding and packaging.

An inspection system may be configured to interact with, monitor, and/or control the converting line. Various sensors and other devices may be arranged adjacent to the converting line may communicate with a controller. As described in more detail below, a convolutional neural network associated with the controller can be utilized to process the communications received from the various sensors. Based on the processing of such communications, the controller may monitor and affect various operations on the converting line. For example, the controller may send various types of control commands to the converter line. In some configurations, the control commands may be in the form of reject commands communicated to the reject system.

It is to be appreciated that the controller and associated convolutional neural network may be configured in various ways. For example, the controller may be in the form of a personal computer (PC), a central processing unit (CPU), a field programmable gate array (FPGA), an application specific integrated circuit (ASIC), or a graphical processing unit (GPU). Preferably a GPU is used. Example hardware with FPGAs may include the National Instruments PCIe-1473R, National Instruments PXIe-1435, National Instruments 1483R with FlexRIO FPGA module, or individual FPGA devices from the Altera Stratix, Altera Cyclone, Xilinx Spartan, or Xilink Vertex series. GPU examples may include devices from NVIDIA platforms such as the Titan series, GeForce GTX 7 through 11 series, Quadro or Jetson series, or from AMD's Radeon HD, R, and RX series.

It is to be appreciated that the controller may also be configured to communicate with one or more computer systems, such as for example, a programmable logic controller (PLC), programmable automation controller (PAC), and/or personal computer (PC) running software and adapted to communicate on an Ethernet/IP network, or using one or more other suitable network protocols, such as USB, FireWire and CameraLink, WiFi or other wireless protocols are also suitable. Some configurations may utilize industrial programmable controllers. The aforementioned configurations may use a personal computer or server running a control algorithm or may be any other device capable of receiving inputs from sensors, performing calculations based on such inputs and generating control actions through servomotor controls, electrical actuators or electro-pneumatic, electrohydraulic, and other actuators. Examples of control algorithms are those from Robovision BV, Technologiepark 80, Ghent University campus, Belgium. Process and product data may be stored directly in the controller or may be located remotely even within the Cloud. In some configurations, and as described in more detail below, the process and product data may include images that are collected by the sensors that can be subsequently utilized as training images when determining convolutional neural network parameters for a convolutional neural network. It is also to be appreciated that the controller may be configured to communicate with various types of controllers and inspection sensors configured in various ways and with various algorithms to provide various types of data and perform various functions.

Since the substrates and components travel in the machine direction through the converting line, the controller may track the advancement of the substrates and components. In some configurations the controller may track the advancement with counts generated by a machine axis that correspond with machine direction positions on substrates and components while advancing though the converting line. In some configurations, the machine axis may be configured as an actual motor that provides count signals to the controller. The controller may utilize rotational speed, time, and/or count data from the machine axis that correspond with the machine direction speed and travel of the substrates and components through the converting line.

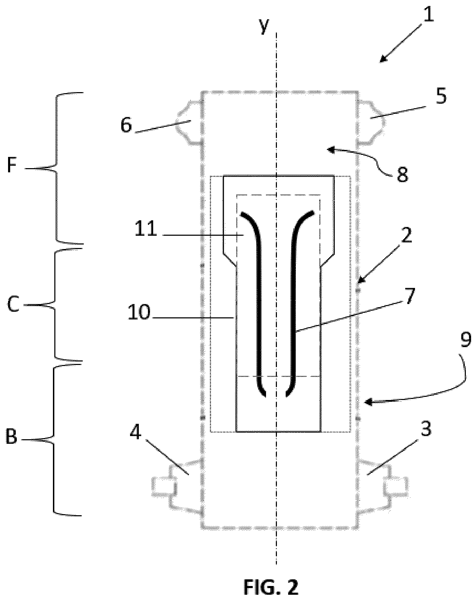

The systems and methods herein may utilize various types of sensors to monitor the substrates and components traveling through the converting line. The sensors may be configured as inspection sensors to monitor various aspects in the substrates and/or components being processed by the converting line or machine. In some configurations, the inspection sensors may be used to assist with the detection of defects within substrates and/or components themselves, such as for example, damage, holes, tears, wrinkles, interrupted channel (as shown in Fig. 2) and the like, and may also be used to assist with the detection of defective assemblies and/or combinations of the substrates and components, such as for example, missing and/or misplaced ears, landing zones, fasteners, and the like. The inspection sensors may be used to assist with the detection of contaminants, such as foreign material, grease, or dirt. As such, inspection sensors may be configured to assist with the detection of the presence or absence of substrates and/or components, and may be configured to assist with the detection of the relative placement of substrates and/or components. As discussed in more detail below, feedback signals from the inspection sensors in the form of inspection parameters are communicated to the controller for processing by an inspection algorithm of the convolutional neural network. In some configurations, for instance, the inspection parameters include images of the base substrate, the auxiliary substrates, discrete components, and so forth. In some configurations, such images can be collected by the inspection sensors without needing to illuminate the area of inspection with light with required specific wavelengths, such as infrared light and/or ultraviolet light. Additionally or alternatively, the inspection parameters can include other forms of data, such as digitized signals, that can be processed by the inspection algorithm of the convolutional neural network.

It is to be appreciated that various different types of inspection sensors may be used to monitor substrates and various components while advancing through the converting line. For example, inspection sensors may be configured as photo-optic sensors that receive either reflected and/or transmitted light and serve to determine the presence or absence of a specific material; metal-proximity sensors that use electromagnetism to determine the presence or absence of a ferromagnetic material; or capacitive or other proximity sensors using any of a number of varied technologies to determine the presence, absence, or thickness of materials. In some configurations, the inspection sensors may also be configured as vision systems and other sub-processing devices to perform detection and, in some cases, logic to more accurately determine the status of an inspected product. Particular examples of such inspection sensors may include Keyence smart cameras, component vision systems or any other vision system software which can run on a PC platform. It should also be appreciated that inspection parameters may be provided from inspection sensors in various forms. In one configuration, inspection parameters may be in the form of images or other types of signals which are processed by the convolutional neural network to determine the presence or absence of a particular defect, feature and/or other component. Such images or signals may also be stored in any suitable data store, such as, for example, a database or in a specified directory on an image server for offline processing in the refinement of the inspection algorithm of the convolutional neural network as described herein.

The systems and methods herein may utilize various types of sensors or data from the controller to monitor the various assembly equipment used in the converting line. Equipment sensors may be configured as process sensors to monitor various aspects of process equipment or operations. In some configurations, the process or equipment sensors may be linear position transmitters, rotary position transmitters, rotational encoders for speed and position feedback, temperature sensors such as RTD elements, pressure and/or vacuum transmitters or vibration sensors. Controller data may be configured as data from drive position or velocity control loops, automatic or operator induced control actions, motor current or power or any other parameter that can be harvested from a controller. Based on the detections of the process sensors, feedback signals from the process sensors in the form of process parameters are communicated to the controller.

The inspection sensors and process sensors, may be connected with the controller through a communication network, which allows the inspection sensors and process sensors to communicate inspection parameters and process parameters, respectively, to the controller. Devices that communicate on the network each may include precision clocks that are synchronized to a master clock within some specified accuracy.

In a preferred embodiment, systems herein do not use time-stamps to provide correlations between processed data, inspection parameters and product feedback. For example a product's unique identifier may be a mathematical sequence. The controller and inspection devices may independently generate the same sequence. When data is stored from varied sources, each piece of data is identified by the product unique identifier rather than a time. This avoids the need of a clock and historian as used in systems today.

Based on the inspection algorithm of the convolutional neural network, the controller may be adapted to send various types of control commands to the converting line, such as for example, speed change commands, reject commands, and shutdown commands. Additionally or alternatively, the control commands can cause various control actions, such as an automatic phase adjustment, an automatic tracking adjustment, a machine maintenance scheduling, and machine stop commands. Such control commands may be based on parameters communicated from various sensors as described above. For example, control commands may be based on images included in the inspection parameters and/or process parameters provided by inspection sensors and process sensors.

In order for the convolutional neural network to process the images, or other data, collected by the sensors, a learning paradigm is preferably utilized to determine convolutional neural network parameters for an inspection algorithm. Once the parameters have been determined, the convolutional neural network can be used in production to produce results independently using the inspection algorithm.

Training can take on many different forms, using a combination of learning paradigms, learning rules, training frameworks, and learning algorithms. The training algorithm can be executed by a processor of a training computing system and/or manually inputted by an operator via a user interface. Once the convolutional neural network is deployed into production, additional learning/training can be utilized (i.e., offline from production) to finetune, calibrate, or otherwise adjust the convolutional neural network parameters. The convolutional neural network can then be updated with the refined parameters to improve performance of the inspection algorithm.

In an example, the images stored in the training image database (herein also referred to as reference database) can include a multitude of images collected from one or more converting lines during production of absorbent articles. The images in the training image database can be tagged or otherwise grouped into training sets (e.g. good or bad; or even by sub-topic e.g. at the component level by utilising for example appropriate metadata), such that each image has a label corresponding to the result that an inspection algorithm is expected to match. For ease of illustration, the images of the training image database are shown to include a set of preprocessed images. As such, each of the images in the training image database can include a labeled component that is of interest for quality control purposes. The labeled component can be, for instance, a feature, aspect, or attribute of the absorbent article that is marked and tagged as such. The labeled components can be components that are desired to be present or components that are undesirable to be present. For instance, the labeled component may mark the fold of the core wrap or may mark a wrinkle, tear, in each image.

Once the training computing system has applied the training algorithm to the images in the training image database, the convolutional neural network parameters can be generated. Example parameters can include, without limitation, the number of convolutional layers, the number of neurons per layer, the number of pooling layers, the weights connecting different layers, number of drop out layers, and so forth. The convolutional neural network parameters can then be implemented as an inspection algorithm of the convolutional neural network associated with a converting line. Based on the determination of the inspection algorithm of the convolutional neural network control commands can be sent to the converting line, as may be needed.

Inspection analysis can be carried out at a pixel level in either gray scale or colored formats.

Converting lines utilizing convolutional neural networks described herein can provide for a variety of different inspection operations to discern consumer noticeable defects during the manufacturing process. Examples of features, flaws, defects, or other issues that can be detected using the systems and methods described herein include holes or tears in substrates, missing components, misplaced components, deformations of components, sufficiency of textures, bond location, bond appearance, and so forth. It can also be used to address potential performance issues e.g. detection of disconnected channels could lead to inadequate liquid distribution performance and fail on lab-test data and thus can be automatically rejected from production without being folded and packaged accordingly.

It is supposed that the present invention is not restricted to any form of realization described previously and that some modifications can be added to the presented example of fabrication without reappraisal of the appended claims.

**Claims**

1. A method for inspecting disposable absorbent articles, the method comprising:

providing a controller comprising an inspection algorithm with a convolutional neural network;
providing a sensor, preferably selected from the group consisting of an optical sensor, thermal sensor, and combinations thereof; the sensor being in data communication with the controller;
providing a reference database, wherein the reference database comprises a plurality of pre-processed images of disposable absorbent articles and/or components thereof that have been labeled with one or more of: a feature or element of the article and/or component thereof; a non-acceptable defect within a feature or element of the article and/or component thereof; an acceptable defect within a feature or element of the article and/or component thereof; an acceptable faultless feature or element of the article and/or component thereof; and combinations thereof; and
the method comprising the steps of:

training the convolutional neural network through a plurality of iterations or epochs;
optionally validating the training, wherein the training step is repeated until a predefined number of iterations is reached or a mean average precision score (MAP) of at least 0.90, preferably from 0.91 to 0.96, is attained according to the formula:

$$MAP = \frac{\sum_{q=1}^{Q} AP(q)}{Q}$$

where Q is the number of queries;

advancing one or more substrates through a converting process along a machine direction to form an array of absorbent articles;

creating a plurality of images of said array of absorbent articles with the sensor;

transmitting the images from the sensor to the controller;

determining at least one or more properties, preferably visual properties, for the labeled component with the inspection algorithm;

cutting the substrate into discrete articles; and

based on the determination of the least one or more properties for the labeled component, executing a control action, wherein the control action is selected from the group of automatic rejection of one or more articles, automatic machine-setting adjustment, a warning signal for machine maintenance scheduling, and a machine stop command.

2. The method of Claim 1, wherein the images comprise at least one of:

at least one image taken with reflected light and at least one image taken with transmitted light for each said article in a sequence of articles;

at least two images taken with transmitted light from respective at least first and second light sources having different wavelength for each said article in a sequence of articles, wherein the first light source emits electromagnetic waves having a wavelength of 100 to 400 nanometers, preferably from 150 to 350 nanometers, and the second light source electromagnetic waves having a wavelength of 450 to 750 nanometers, preferably from 500 to 700 nanometers; and

at least two images taken with transmitted light by a first sensor, typically being an optical sensor, and a second sensor, typically being a thermal sensor, respectively for each said article in a sequence of articles.

3. The method of any of the preceding Claims, wherein the pre-processed images are automatically labeled by: said inspection algorithm after the training step or by a further inspection algorithm.

4. The method of any of the preceding Claims, further comprising the step of automatically feeding at least a portion of captured images to the reference database to increase the number of pre-processed images for the inspection algorithm to use in the determination step, preferably wherein said at least portion comprises images that are different from existing images in the reference database prior to the feeding step.

5. The method of any of the preceding Claims, wherein the pre-processed images are tagged with metadata comprising information about at least one of: the image source type such as whether reflected or transmitted light was used or whether other non-visual imaging sources e.g. heat were used; absorbent article type such as a diaper or pant; element or component of an absorbent article such as an elastic ear panel or absorbent core; a command prompt indicator such as qualifiers selected from acceptable or not acceptable; and combinations thereof; and preferably wherein the reference database is arranged to cluster said images based on said metadata; and preferably wherein the inspection algorithm automatically compares one or more captured images, taken in the step of creating a plurality of images of said array of absorbent articles with the sensor, with the most similar images in the reference database based on said metadata.

6. The method of any of the preceding Claims, wherein the training step comprises the step of creating a pipeline that is further transmitted to the, preferably trained, inspection algorithm or further inspection algorithm for pre-processing prior to being transmitted to the reference database.

7. The method of any of the preceding Claims, wherein the controller comprises a graphics processing unit and/or a central processing unit.

8. The method of any of the preceding Claims, wherein the labeled component comprises any of a contaminant and an undesirable transformation of the article, preferably wherein the undesirable transformation of the article comprises any of a mis-positioned component or fold, a hole, a tear, and a wrinkle.

9. The method of any of the preceding Claims, wherein the determination step is carried out over substantially each entire image without dividing each image into a plurality of segments to virtually segment the absorbent article or substrate(s) into a plurality of virtual segments for applying inspection parameters.

10. The method of any of the preceding Claims, wherein the one or more properties for the labeled component are selected from: an angular orientation of the labeled component; the absence of the labeled component; a texture or contrast level of the labeled component; a total number of the labeled components; a deformation of the labeled component.

11. The method of any of the preceding Claims, wherein the articles are selected from the group consisting of diapers, pants, pantiliners, briefs, and sanitary napkins.

12. The method of any of the preceding Claims, further comprising a step of storing process data or product data remotely, preferably on the Cloud.

13. The method of any of the preceding Claims, further comprising a step of masking the labeled component in the images by analyzing the images with a first inspection algorithm.

14. The method of any of the preceding Claims, wherein the absorbent articles are substantially translucent.

15. The method of any of the preceding Claims, wherein the one or more properties comprise any of: the absence or presence of discontinuities within a channel (7) free of absorbent material within the absorbent core of said articles; positioning of a fold of one or more core wrap layers within the absorbent core or absorbent assembly; presence or absence of a fold within an elastic side panel (3,4) said fold positioned between a topsheet (8) and a backsheet (9) of the absorbent article wherein said topsheet and backsheet sandwich the absorbent core or absorbent assembly therebetween; positioning of an acquisition distribution layer with respect to a perimeter of an absorbent core or absorbent assembly of the article; positioning of an absorbent core or absorbent assembly with respect to a perimeter of a chassis (2) of the absorbent article (1).

16. A method for automatically inspecting one or more components of an absorbent article manufacturing machine or disposable absorbent articles made with said manufacturing machine, the method comprising:

   providing a controller comprising an inspection algorithm with a convolutional neural network;
   providing a sensor, wherein said sensor is an acoustic sensor, preferably a microphone;
   providing a reference database, wherein the reference database comprises a plurality of pre-processed sound recordings of one or more components of the absorbent article manufacturing machine when running or in-operation, preferably said components comprising one or more knives during a cutting step, one or more rotating elements during a conveying step, and/or one or more joining elements, preferably selected from adhesive applicator(s) and sonotrode(s), during a joining or lamination step; or one or more parts of disposable absorbent articles as they are processed by said manufacturing machine; that have been labeled with one or more of: a sound corresponding to a failure or rupture of said one or more components or one or more parts; a sound corresponding to a damage of said one or more components or one or more parts; a sound corresponding to a correct or acceptable running of said one or more components or one or more parts; and
   the method comprising the steps of:

      training the convolutional neural network through a plurality of iterations or epochs;
      optionally validating the training, wherein the training step is repeated until a predefined number of iterations is reached or a mean average precision score (MAP) of at least 0.90, preferably from 0.91 to 0.96, is attained according to the formula:

$$MAP = \frac{\sum_{q=1}^{Q} AP(q)}{Q}$$

      where Q is the number of queries;
      advancing one or more substrates through a converting process along a machine direction to form an array of absorbent articles;
      creating a plurality of sound recordings of said one or more components of the absorbent article manufacturing machine when running or in-operation, or one or more parts of disposable absorbent articles as they are processed by said manufacturing machine, with the sensor;
      transmitting the sound recordings from the sensor to the controller;

determining at least one or more acoustic properties of the labeled component or part with the inspection algorithm;

cutting the substrate into discrete articles; and

based on the determination of the least one or more acoustic properties for the labeled component or part, executing a control action, wherein the control action is selected from the group of automatic rejection of one or more articles, automatic machine-setting adjustment, a warning signal for machine maintenance scheduling, and a machine stop command.

17. A method according to Claims 1 to 15 comprising the step of further providing an acoustic sensor, preferably a microphone, and the method comprising the steps of Claim 16.

FIG. 1

FIG. 2

C

S

Reference
Database

User Interface

MD

**FIG. 3**

Europäisches
Patentamt

European
Patent Office

Office européen
des brevets

## EUROPEAN SEARCH REPORT

**Application Number**

EP 21 17 0230

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/353348 A1 (KAWKA PAUL ANTHONY [US] ET AL) 13 December 2018 (2018-12-13) * claims * | 1-17 | INV. A61F13/15 G05B19/418 |
| A | US 2021/026338 A1 (YATI ARPIT [IN] ET AL) 28 January 2021 (2021-01-28) * the whole document * | 1-17 | |
| A | EP 2 437 706 B1 (PROCTER & GAMBLE [US]) 25 March 2020 (2020-03-25) * the whole document * | 1-17 | |

TECHNICAL FIELDS
SEARCHED      (IPC)

A61F
G05B

The present search report has been drawn up for all claims

| Place of search | Date of completion of the search | Examiner |
|---|---|---|
| Munich | 14 October 2021 | Hoff, Céline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 21 17 0230

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2021

| Patent document cited in search report | | Publication date | Patent family member(s) | | Publication date |
|---|---|---|---|---|---|
| US 2018353348 | A1 | 13-12-2018 | US 2018353348 A1 | | 13-12-2018 |
| | | | US 2018357756 A1 | | 13-12-2018 |
| | | | US 2020222246 A1 | | 16-07-2020 |
| | | | US 2020222247 A1 | | 16-07-2020 |
| US 2021026338 | A1 | 28-01-2021 | TW 202119455 A | | 16-05-2021 |
| | | | US 2021026338 A1 | | 28-01-2021 |
| | | | WO 2021021533 A1 | | 04-02-2021 |
| EP 2437706 | B1 | 25-03-2020 | EP 2437706 A1 | | 11-04-2012 |
| | | | US 2010305740 A1 | | 02-12-2010 |
| | | | US 2012150336 A1 | | 14-06-2012 |
| | | | US 2014200701 A1 | | 17-07-2014 |
| | | | US 2016342153 A1 | | 24-11-2016 |
| | | | US 2019271972 A1 | | 05-09-2019 |
| | | | WO 2010141547 A1 | | 09-12-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10331113 B